# EUROPEAN PATENT SPECIFICATION

(11) **EP 4 069 350 B1**
(45) Date of publication and mention of the grant of the patent: **13.12.2023**
(21) Application number: 19842886.4
(22) Date of filing: 22.12.2019
(51) Int. Cl.: A61N 1/16, A61N 2/02

(54) **A WIDE-RANGING NEUTRALIZER OF ELECTROMAGNETIC WAVES WHICH ARE HARMFUL TO HUMANS**
BREITER NEUTRALISATOR FÜR ELEKTROMAGNETISCHE WELLEN, DIE FÜR MENSCHEN GEFÄHRLICH SIND
VASTE NEUTRALISEUR D'ONDES ÉLECTROMAGNÉTIQUES QUI SONT NOCIVES POUR LES ÊTRES HUMAINS

(30) Priority: 04.12.2019 RS MP20190076 U
(43) Date of publication of application: 12.10.2022
(73) Proprietor: Bijelovic, Dorde, Beograd 11090 (RS)
(72) Inventor: Bijelovic, Dorde, Beograd 11090 (RS)
(74) Representative: Vlahovic, Slobodan
(86) International application number: PCT/IB2019/061256
(87) International publication number: WO 2021/111178

(56) References cited:
- WO-A1-03/006105
- WO-A1-2004/103464
- RS-U- 1 393

## Description

### THE AREAS OF TECHNOLOGY TO WHICH THE INVENTION RELATES

The invention in question falls, in general, under the category of devices which are utilized to neutralise the harmful effects of electromagnetic radiation and more specifically, it is a neutralizer of harmful electromagnetic radiation which in turn also brings harmony to the biofield of a human body as well as its surroundings.

According to the International patent classification (IPC, IntCL7), the patent has been filed under and marked with the classification symbol A61N 1/16 which relates to the protection from or neutralisation of harmful radiation.

Since this device is at the same time one which contains solenoids for creating electromagnetic waves of a certain frequency, it is also labelled with a secondary classification symbol A61N 2/02 which includes the use of a magnetic field produced by a coil, including single turn loops or electromagnets.

### THE TECHNICAL PROBLEM

The technical problem which is solved by the patent is as follows: how to find a solution to construct a device which achieves a permanent protection from and neutralisation of harmful electromagnetic radiation, whose principle of operation is based on coaxial looped solenoids created from the noble metals which create a potential difference between the neutralizer elements and form a weak high frequency electrical field which effectively cancels the effects of the low frequency electro-magnetic waves by behaving both as a regular oscillator circuit whose radiation, when in close proximity to harmful radiation, using induction and self-induction creates a harmonisation of the area imediatelly around the user by generating electromagnetic radiation useful to humans, while being consistent, constantly charged by the thermal energy of the user, it does not get warm, it has a wider range of protective effects and has no negative consequences of usage.

### THE CURRENT STATE OF TECHNOLOGY

Radiation penetrates all cells of the human body and causes physical and psychic disorders. On the physical level the radiation causes a disruption of the immune system and the metabolism, which, especially during long term affects of the harmful radiation, can lead to many illnesses, included the most serious ones. On a psychological level the harmful radiation leads to the occurance of depression, agressivenes, loss of concentration, malaise and hypersensitivity to stress. In a technical sense all of the above mentioned negative occurences are a consequence of the deformed electromagnetic field which creates disharmony within the human biofield.

It is a well-known fact today that a human being is, fundamentally, a biological and energetic system which is in constant energetical and informational interaction with its surroundings. It means that he is continually exposed to the influences of different physical fields: gravitational, electrostatic, magnetic, electromagnetic and a number of other harmful waves.

In addition to the harmful, there is also a beneficial form of radiation which positively affects the human bioenergetic field and it serves as a guide for those who wish to create inventions which will strive to get as close as possible to the beneficent radiation of Nature, with positive effects on the human health and psyche, relaxing him and getting rid of harmful energy flows, healing him.

Not so long ago the positive influence of air ionisers was challenged and today any well equipped office space would be unimaginable without one, as well as conference and other work spaces, especially medical institutions, operating theatres or emergency rooms. Today the positive effects of neutralizers are widely accepted and they are therefore frequently in use in the airforce, submarines and other workplaces which are full of harmful radiation.

By researching the available patent documentation and other technical documentation from the area in question and the available technical and promotional literature, the following can be found:
In the Yugoslav journal number 48657 published on 10.07.1998 under the title "Inductive absorptional insert for removing electric and magnetic radiation", a device was described and shown, a device around whose insert two standard coils are looped. Both are looped around the same central axis in the same direction, one facing forwards and the other backwards. The ends of the conductors are connected by a conductive connection, while the coils go around the conductor. The device functions in such a way that the two loops absorb the energy of the electric and magnetic fields and use it to create currents in the coils. The induced current in one winding is the same as the current in the other winding, with these opposing currents cancelling each other out. The end result of the device function is that energies of the electrical and magnetic fields are used up.

In the German application of patent DE 3220565 (A1) published on 01.12.1983. a device was described which creates a reverse electromagnetic field to the pathogenic electromagnetic field. That device only solves one problem - that of the electrical or that of the magnetic fields together with their consequences, and for its functioning it requires a constant supply of electricity.

In the German application of patent DE 3341756 (A1) published on 30.05.1985. a device was described and shown a bed cover with metal fibres for protection from electrical and magnetic fields, whose effects are narrowly limited to the area just around the bed.

In the Yugoslav application of patent YU89992 there was a description of a bioenergetic re-equaliser which was presented as an energy transmitter for the absorption and elimination of all harmful and destructive radiation to the human body. Also in the Yugoslav applications P-2154/90 and P-2155/90 there was a description of a "Neutralizer of harmful radiation" in the form of a ring with nails mounted in a multicomponent mass embedded in the box as well as a "Harmful radiation arrester" with a socket connected by a coil cable housed in a mass contained in a box.
- In the international application PCT/YU01/00019 WO 03006105 published on 23.01.2003. and the granted small patent YU 413 MP which is in its construction closest to the solution of the present application, for the oscillator circuits to generate their own oscillations and by which, at the same time, the induction of external harmful radiation is converted into complex alternating currents of lower frequencies, it is accomplished by means of three coupled cross-wound solenoids in each of the two chambers of the body, intersected by the rod core. Each solenoid individually is made up of equal threads, which results in each solenoid having a fixed inductance, with the highest possible capacitance being achieved when the tip of the rod core is at the greatest distance from the lids, either on one side or the other, and in its essence it is very small. This means that each solenoid with a nucleus forms a range of frequencies around the three basic frequencies and these frequency bands do not overlap. In this way, only parts of the harmful spectrum of electromagnetic radiation of technical appliances and devices are neutralized from the human environment.
- In the Serbian granted patent number RS 49631 B of Radovic Savo a device was shown for the neutralisation of the wide spectrum of harmful electromagnetic radiation which consists of two identical cylinders with cylindrical chambers interconnected by a cylindrical channel through a cylindrical ring through which a rod core with a conical solenoid is threaded, where the novelty was that the rod core at both ends ends with disks, with the solenoids being conical. This construction allows significantly increased capacitance on the one hand, ie. variable values of continuous inductance, thus creating continuous resonant circuits to achieve self-resonance or induction of external harmful electromagnetic radiation currents, and so thus this radiation is converted to lower frequencies in the device or, when self-resonating, is propagated from the device and cancels out external harmful radiation. Such a device undoubtedly has a positive neutralizing effect, but there is the question of its efficiency, since all the outer edges of the device are at the same distance from the solenoids located at its centers, so that they produce a constant magnetic field of a certain electromagnetic spectrum, which brings into question its claimed that it is a wide-spectrum device.
- In the Sebian granted small patent RS 1393 U, published 31. 12. 2014. the device inventor presented a device called "Vacuum Neutralizer for Electromagnetic Radiation and a Schumann Frequency Generator" consisting of a hollow body with cylindrical lids providing a low vacuum of 100kPa to which the center rod, solenoids, magnets, oscillators and vacuum valve are attached, and which, by their operation, cause only the induction and formation of eddy (Foucault) currents, which create the effect of neutralizing the surrounding harmful electromagnetic radiation. This device has proven to be effective, but due to the use of vacuum, the whole structure is complicated and, given the small dimensions, practice has shown certain problems related to the maintenance of vacuum and durability over a long period of time.

### REVEALING THE ESSENCE OF THE INVENTION

The essence of the invention lies in a broad-band neutralizer of electromagnetic radiation harmful to humans, shaped as a hollow cylinder and is composed of the brass housing, at the ends of which two symmetrical cylinders with equal chambers of circular cross-sections are constructed, whereby a round iron core is axially passed through the center of the neutralizer onto which, in each chamber, three solenoids are coaxially placed, positioned in such a way that the smallest diameter solenoids are orientated towards the middle of the neutraliser and the widest solenoids lean on the plates which on both sides hermetically seal the free ends of the housing.

The essence of the invention is also contained in the choice of materials which make up the body of the neutralizer, as well as the type and thickness of the wire from which the solenoids are made, as well as the thickness of the core and the layout of the annular external lateral reinforcements created, in this invention, in such a way that they significantly widen the range of the self-oscillation frequency, as well as externally induced osciallations, thus allowing for absorption of harmful radiation to be much more efficient and in a much wider frequency range.

**The invention innovation** is also contained in the shape of the neutralizer, the proportion of the component parts and the positioning of the solenoids in such a way that the coils partially overlap, which creates a more comprehensive absorption of harmful radiation from all directions and at the same time creates a better spatial redistribution of most of the harmful radiation from the environment to the neutralizer.

What makes this neutralizer different in comparison to similar radiation absorbers of other inventors is the choice of the wire thickness, the arrangement of the solenoids and how they are wound, which is done in such a way that the total inductivity of the solenoid is continually changing, with a significant increase in the maximum value of the also variable capacitance, which all in all significantly affects the efficiency of the neutralizer in question.

Another innovation of this invention is that the power supply solution for the neutralizer is permanent, by the heat exchange between the device and the human body, which has significantly simplified construction.

**A further innovation** is also found in that the solenoids in the chambers of the housing are under normal air pressure, which avoids the solutions of the past which have relied on vacuum, which has historically made both production and maintenance more complicated and was also reflected in the price. Most importantly, this type of innovative construction has avoided problems associated with the warming of the neutralizer which sees a decrease in efficiency at higher temperatures.

Except the above mentioned, the invention does also have the following advantages over the existing technological solutions, of which only the main ones are listed here and they are:
- it is easy to produce and because of its simple construction requires no special servicing or maintenance;
- because of its great conductivity with respect to the human body, it is very efficient in taking a great part of the harmful radiation energy which, in large part, is spent in the ohmic resistance of the oscillatory circuits;
- thanks to its construction, especially due to the three-stage coaxial solenoids, it possesses a much wider range of resonant frequencies than the existing absorbers and thus neutralises a wider range of technically harmful radiation with a greater degree of efficiency;
- because of its esthetic properties it can also be used as part of decorations and jewlery and is therefore additionally attractive to the younger population.

### A SHORT DESCRIPTION OF THE DRAWINGS

With the aim of easier understanding of the invention, the author refers to the enclosed application drawings by way of example where:
- Figure 1. represents a schematic representation of the vertical cross-section the neutralizer in question;
- Figure 2. represents the side on view of the neutralizer in question;
- Figure 3. represents the view from above of the neutralizer in question;
- Figure 4. represents the view from below of the neutralizer in question;
- Figure 5. represents the axonometric view of a circular plastic plate with an electronic oscillator.

### A DETAILED DESCRIPTION OF THE INVENTION

Looking at the enclosed drawings it is easy to see that the neutralizer 1 of the electromagnetic radiation harmful to the human body is composed of: a housing 2 shaped like a hollow roller whose ends are symmetrically formed by cylinders 3, 4 with chambers 5, 6, a ring coupler 14 with a tubular axial cavity 7 through which the iron core is axially drawn 8 onto which two assemblies of solenoids are drawn 9, 10, lids 11, 12, the electronic oscillator 21, a cylindrical magnet 19 and a brass circular plate 13.

The housing 2 of the neutralizer, made of brass, is in the form of a hollow roller whose ends are designed as cylinders positioned opposite one another 3, 4 integrally connected by a ring coupler 14 on which a concave, rounded groove 35 is made in the middle. The cylinders 3, 4 house the prismatic chambers 5, 6 which are of a circular cross-section, with hollow conical ends 28, 29 which are equal and connected to one another through the center of the coupling 14, by the axially formed tubular cavity 7. The ends of the cylinders 3, 4 are the tubular ends 15, 16 on whose inner sides are threaded threads 36 which enable the separable covers 11, 12 to be afixed and which are made of the same material as the housing 2. On the outside of the lid 11, made as a two-part cone whose lower segment 17 is a hollow tube with a thread threaded on the outside 36, in accordance with the thread 36 on the tubular end 15, while its second, free end 18 is formed in a cone shape with the tip facing in the opposite direction to the center of the housing 2. In segment 17, a cylindrical magnet 19 is inserted, the bottom of which is supported by a circular plate 13 resting on an indent made on the housing 2, while to its upper surface is glued a thin circular plate 20 of plastifix with an electronic oscillator 21 which is, just for the sake of proof of feasibility of the invention, shown as a modified form of the Pierce osciallator with a minimal number of components (a digital inverter, two resistors, two capacitors and a quartz crystal), which, despite its simple construction, has exceptional stability and is designed to oscillate at the frequency of 7.8 Hz (Schumann resonance).

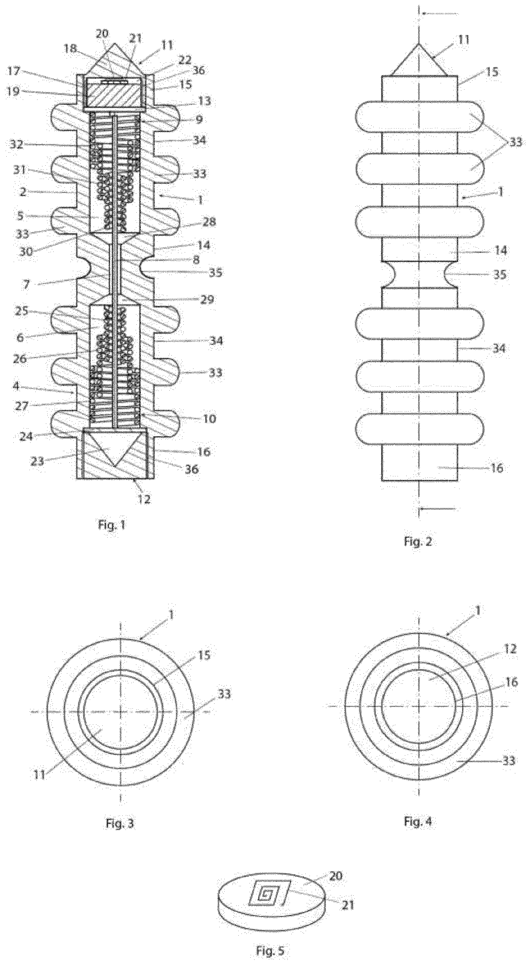

It can be easily seen from Figure 1 that an axial cavity 7 is drawn in the center of the connector 14 through which an iron core 8 of circular cross-section is drawn, so that its ends are symmetrically rested along the centers of the chambers 5, 6.

To the tubular end 16 of the housing 2, a lid 12, is afixed using a laterally threaded thread 36 and is shaped like a roller at the end of which is mounted a cone-shaped resonant cavity 23 oriented towards the center of the neutralizer 1, with a base facing the chamber 6, which, due to its conical shape, increases the conductivity of the electromagnetic waves from the environment directing them to the assembly of solenoid 10 where they are neutralized through the ohmic resistance of the oscillatory circuit consisting of solenids 25, 26, 27, the inner side of the cylinder 3 and the iron core 8. The opening at the end of the chamber 6 is closed by a circular brass plate 24 resting on an indent mounted on the housing 2 and secured by the lid 12, allowing for easy insertion or removal of the solenoids 25, 26, 27 as needed.

Onto the core 8, made of soft iron, inserted to more efficiently generate Eddy currents and amplify the magnetic fields, are fixed opposite each other the solenoid assemblies 9, 10 consisting of solenoids 25, 26, 27, 30, 31, 32 done in such a way that directly onto the iron core 8, in the continuation of the cavities 28, 29, are first drawn solenoids 25, 30 of the smallest diameter, and then patially overlapping, the intermediate sized solenoids 26, 31 and finally, so that they overlap by half along their lengths, solenoids 27, 32 of the largest diameters are also coaxially fixed. The assemblies of solenoids 9, 10 are of such longitudinal dimensions that their free ends are supported by circular plates 13, 24. It should be noted that this construction of solenoid assemblies 9, 10 came about after long experiments and measurements which found that just in such a way co-axially positioned solenoids showed the best results in protection against a large number of harmful electromagnetic radiation. All solenoids are made of silver wire 99% pure, with solenoids 25, 30 having a wire thickness of 0.1 mm, solenoids 26, 31 0.2 mm, and solenoids 27, 32 0.3 mm. This arrangement and method of making the solenoids avoided the technical solution according to which the solenoids were movable in order to extend the frequency range, which showed a number of shortcomings in terms of durability, maintenance, and even efficiency of operation.

On the outer sides of the walls of cylinders 3, 4 are integrally formed three identical radial annular reinforcements 33, positioned in such a way that the outer edges of the end reinforcements coincide with the outer edges of cylinders 3, 4. The reinforcements 33 are parallel, with equal distances 34, rounded outer edges and are designed to increase the capacitance of the oscillator circuit of the neutralizer by a combination of convex and concave surfaces, which is significant for the induction and self-induction processes, thus directly affecting the efficiency of the neutralizer operation. The mode of operation of the neutralizer in question is the closest to the mode of operation of a linear oscillator circuit and occurs in that the potential difference between the housing and the solenoid circuits creates a weak highfrequency electric field that nullifies the effects of harmful natural and technical radiation of lower frequency, which were found to, during a prolonged exposure of the human body to them, due to the absorbed energy, become transformed into another form of energy, and thus impair the potential of healthy cells leading to their deharmonization, which is the cause of numerous diseases. The application of the present invention neutralizes the effects of harmful electromagnetic radiation, which eliminates or significantly reduces physical and mental disorders and allows the unhindered flow of bioenergy throughout the body, having a positive impact on its vitality and regeneration.

Unlike similar devices designed to have their oscillator circuits housed in a vacuum, the solenoid assemblies 9, 10 of the neutralizer in question are housed in chambers 5, 6 which are under normal atmospheric pressure, thus eliminating the problem of rapid heating which is otherwise present on all vacuum neutralizers. It should be emphasized that it has been reliably established that the efficiency of the neutralizer decreases dramatically with increasing temperature, that is, the results obtained with devices in which the solenoids are in the air are significantly better than when they are stored in a vacuum, precisely because in the second case, they heat up faster, thus significantly reducing their protective properties.

The assemblies of solenoids 9, 10 suspended on the iron core 8 are designed to magnetize it permanentlee. This process, which is successive but a certainty, is known as conditioning, and is such that a specific form of magnetic domains is thus obtained in the magnetic iron core 8, thus generating electromagnetic waves of different frequencies in the solenoid, which are then reflected into the surrounding space via the ring reinforcements 33 and the cone 18. The calculation of the length and thickness of the iron core 8, as well as the number of windings of the solenoid 9, is designed so that the excitation current during conditioning is such that the magnet "remembers" the frequency of the Schumann resonances, which causes the neutralizer to continuously exert its positive effect on the surrounding space. The effect of this neutralizer 1 is based on the prevention of the harmful effects of magnetic fields and electromagnetic fields on the carriers of ionic charges in the bloodstream and biofluids, with the simultaneous permanent elimination of induced Faraday eddy currents, which are well known today to cause a large number of cellular and tissue changes in the membranes and the interior of the cells, which in living organisms lead to numerous pathological problems and various types of damage to the organism. It is important to emphasize here that the essence and operation of this device, just like Tesla's magnetic vacuum amplifiers and its purple protective tiles are not fully known.

By carefully monitoring the workings of the neutralizer in question, it has been proven beyond doubt that positioning of the solenoid assemblies 9, 10 in the air-filled chambers 5, 6, improved the constancy of the neutralizer's functioning, thereby completely avoiding the failings observed with similar vacuum neutralizers.

### METHOD OF INDUSTRIAL OR OTHER APPLICATION OF THE INVENTION

The industrial fabrication of the present invention is absolutely possible in factories for the manufacture of electronic devices and medical equipment, and even in well-equipped workshops for the manufacture of home electrical appliances, on the basis of technical documentation which experts in the field in question can do easily using the descriptions and designs in this application.

The usage of the present invention is particularly recommended to all persons with a pronounced sensitivity to geopathogenic radiation as well as to the harmful radiation caused by the effects of electric appliances, substations, electric tram networks, trolleybus and electric trains and their overhead or track power supplies, as well as in the case of amplified electromagnetic radiation coming from space.

The use of the device is also recommended in surgery rooms, intensive care rooms and ICUs, and in general in hospitals and rooms requiring special treatment of human organisms for their faster recovery and relief. It is to be noted that the positive effects of the neutralizer are evident in humans, plants, as well as animals.

It is especially advisable to use the neutralizer in electrically powered vehicles bearing in mind the electromagnetic radiation present due to the operation of the vehicle's propulsion motors, as well as to the users of the 5G network, who are exposed to the effects of its transmitters over a long period of time.

It is advisable to use the neutralizers when staying or working in buildings that have been designed as Faraday cages for various reasons.

## Claims

1. A wide frequency neutralizer of electromagnetic waves which are harmful to humans, consisting of: a housing (2), cylinders (3, 4) with chambers (5, 6), a tubular axially extending cavity (7) through which an axially iron core (8) is drawn onto which two solenoid assemblies 9, 10), lids (11, 12) and brass circular plates (13, 24) are provided, wherein the cylinders (3, 4), integrally connected by a ring coupling (14) with a centrally concave rounded groove (35), with the cylinders (3, 4) having mutually equal chambers (5, 6) of circular cross-section with hollow conical ends (28, 29), connected through the center of the coupler (14) by the tubular axially extending cavity (7), and by the fact that the ends of the cylinders (3, 4) having the form of tubular ends (15, 16) with threads (36) cut in on the inside, by means of which the lids (11, 12) made of the same material as the housing (2), are removably fixed, and by having on a lower segment (17) of the lid (11), made as a two-piece cone, threads (36) cut in on the outside, fitting the thread (36) on the tubular end (15), while the other, free end (18) of the cylinder (4) is shaped like a cone with the top facing the opposite of the center of the housing (2), and by the fact that a cylindrical magnet (19) is inserted into the lower segment (17) with the bottom of one side resting on the brass circular plate (13) inserted into the groove on the housing (2), while a thin circular plate (20) of plastifix on which a pure liquid silver electronic oscillator is created (21) which oscillates at 7.8Hz, fixed by gluing to an upper surface of the cylindrical magnet (19) and by having first, second and third solenoids (25, 26, 27, 30, 31, 32) made of 99% pure silver wire coaxially slid on the iron core (8), the first solenoids (25, 30) having a wire thickness of 0.1 mm, the second solenoids (26, 31) having a wire thickness of 0.2 mm, the third solenoids (27, 32) having a wire thickness of 0.3 mm, by having in the continuation of the cavity (29) the first solenoids (25,30) and the second solenoids (26, 31) partially overlapping the first solenoids (25, 30), and the third solenoids (27, 32) overlapping the second solenoids (26, 31) by half the length of the second solenoids (26,31), with the solenoid assemblies (9, 10) being of such dimensions that their free ends are supported by the brass circular plates (13, 24).

2. The wide frequency neutralizer, according to claim 1, wherein the solenoids (25, 26, 27, 30, 31, 32) in the chambers (5, 6) are under normal atmospheric pressure.

## Patentansprüche

1. Breitfrequenter Neutralisator derjenigen elektromagnetischen Wellen, die eine schädliche Wirkung auf den menschlichen Körper haben, bestehend aus: einem Gehäuse (2), Zylindern (3, 4) mit Kammern (5, 6), einem röhrenförmigen, axial verlaufenden Hohlraum (7), durch den ein axialer Eisenkern (8) gezogen wird, auf den zwei Magnetbaugruppen (9, 10), Deckel (11, 12) und eine kreisförmige Messingplatte (13) vorgesehen sind, **dadurch gekennzeichnet, dass die Zylinder (3, 4) durch eine Ringkupplung (14) einstückig mit der Stelle verbunden sind, an der** in der Mitte eine konkave, abgerundete Nut (35) ausgebildet ist, und dass sie durch die Mitte der Kupplung (14) durch einen axial ausgebildeten rohrförmigen Hohlraum (7) Kammern (5, 6) mit kreisförmigem Querschnitt mit hohlen konischen Enden an den Enden (28, 29) bilden, die gleich und miteinander verbunden sind, und dadurch, dass die Enden des Zylinders (3, 4) rohrförmig (15, 16) sind, auf die innen (36) die Gewinde aufgefädelt sind, mit denen die Deckel (11, 12) ablösbar und aus dem gleichen Material wie das Gehäuse (2) gesichert sind, und ebenfalls am Deckel (11) als zweiteiliger Konus ausgebildet sind, dessen unteres Segment (17) ein Hohlrohr mit außen eingefädelten Gewinden ( 36) identisch mit den Gewinden am rohrförmigen Ende (15), während sein anderes, freies Ende (18) kegelförmig geformt ist, wobei die Oberseite der Mitte des Gehäuses (2) gegenüberliegt, und durch Einsetzen eines zylindrischen Magneten (19) in das Segment (17), dessen Boden auf einer kreisförmigen Platte (13) ruht, die auf einer Nut im Gehäuse (2) ruht, während an seiner Oberseite durch Aufkleben eines kreisförmigen Platte (20) aus Plastifix, auf der ein reiner flüssiger elektronischer Oszillator (21) erzeugt wird, der mit 7,8 Hz schwingt (Schumann-Resonanz) und der Eisenkern (8), auf den koaxiale Magnetspulen (25, 26, 27, 30, 31, 32) gezogen sind, die aus 99 % reinen Silberdrähten und verschiedenen Dicken von 0,1-0,3 mm bestehen, die so hergestellt sind, dass sie direkt auf den Eisenkern (8) aufliegen, In der Fortsetzung des Hohlraums (29) sind zunächst Spiralmagnete (25, 30) kleinster Durchmesser und dann diese teilweise überlappend, die Mittelmagnete (26, 31) und schließlich, so daß sie sich um die halbe Länge überlappen, auch koaxial feststehende Magnete (27, 32) mit größtem Durchmesser eingezogen, wobei die Baugruppen Magnete (9, 10) von solchen Abmessungen sind, daß ihre freien Enden von kreisförmigen Platten (13, 13, 24).

2. Breitbandneutralisator der elektromagnetischen Wellen mit schädlicher Wirkung auf den menschlichen Körper, wie in der obigen Forderung 1 dargelegt, **dadurch gekennzeichnet, dass** die Magnetspulen (25, 26, 27, 30, 31, 32) in den Kammern (5, 6) unter normalem atmosphärischem Druck stehen.

## Revendications

1. Un neutralisant à large fréquence des ondes électromagnétiques ayant un effet délétère sur le corps humain, constitué: un boîtier (2), des cylindres (3, 4) avec des chambres (5, 6), une cavité tubulaire extensible axialement (7) à travers laquelle est tiré un noyau axialement en fer (8) sur lequel sont prévus deux solénoïdes (9, 10), des couvercles (11, 12) et une plaque circulaire en laiton (13), **caractérisés en ce que les** cylindres (3, 4) intégralement reliés par un coupleur annulaire (14) à l'endroit où se forme une rainure concave arrondie (35) au milieu, et ils forment des chambres (5, 6) de section circulaire avec des extrémités coniques creuses aux extrémités (28, 29), qui sont égales et reliées les unes aux autres, à travers le centre de l'attelage (14) par une cavité tubulaire formée axialement (7), et par le fait que les extrémités du cylindre (3, 4) sont tubulaires (15, 16) sur lesquelles les filetages sont filetés à l'intérieur (36) par lesquels les couvercles (11, 12) sont fixés de manière à pouvoir être détachés et fabriqués à partir du même matériau que le boîtier (2), et sont également construits sur le couvercle (11) comme un cône en deux parties dont le segment inférieur (17) est un tube creux avec des filetages filetés à l'extérieur ( 36) identique aux filetages de l'extrémité tubulaire (15), tandis que son autre extrémité libre (18) a la forme d'un cône dont le haut est orienté vers l'opposé du centre du boîtier (2), et par l'insertion d'un aimant cylindrique (19) dans le segment (17), dont le fond repose sur une plaque circulaire (13) reposant sur une rainure dans le boîtier (2) tandis que sur sa surface supérieure est fixé par collage d'une plaque (20) de plastifix sur laquelle est créé un oscillateur électronique en argent liquide pur (21) oscille à 7,8 Hz (résonance Schumann) et le noyau de fer (8) sur lequel sont dessinés des solénoïdes coaxiaux (25, 26, 27, 30, 31, 32) constitués à 99 % de fils d'argent pur et de différentes épaisseurs de 0,1-0,3 mm créés de telle sorte que directement sur le noyau de fer (8), Dans la suite de la cavité (29) sont d'abord dessinés des solénoïdes enroulés (25, 30) de plus petit diamètre puis les chevauchant partiellement, les solénoïdes du milieu (26, 31) et enfin, de sorte qu'ils se chevauchent de la moitié de la longueur, également des solénoïdes fixés coaxialement (27, 32), du plus grand diamètre, les assemblages étant des solénoïdes (9, 10) de dimensions telles que leurs extrémités libres sont soutenues par des plaques circulaires (13, 24).

2. Neutralisant à large bande des ondes électromagnétiques ayant un effet néfaste sur le corps humain, tel que défini dans la demande 1 ci-dessus, **caractérisé en ce que les** solénoïdes (25, 26, 27, 30, 31, 32) dans les chambres (5, 6) sont soumis à une pression atmosphérique normale.
